# EUROPÄISCHE PATENTANMELDUNG

(11) **EP 2 332 513 A2**
(43) Veröffentlichungstag der Anmeldung: **15.06.2011**
(21) Anmeldenummer: 10186779.4
(22) Anmeldetag: 07.10.2010
(51) Int. Cl.: A61K 8/04, A61K 8/35, A61K 8/37, A61K 8/49, A61K 8/895, A61Q 17/04

(54) **Sonnenschutzzusammensetzungen mit verbessertem Lichtschutzfaktor**

(30) Priorität: 16.10.2009 DE 102009045753
(71) Anmelder: Henkel AG & Co. KGaA, 40589 Düsseldorf (DE)
(72) Erfinder: Janßen, Frank, 41470, Neuss (DE); Dickhof, Susanne, 41748, Viersen (DE)

(57) **Zusammenfassung**

Gegenstand der vorliegenden Anmeldung sind kosmetische Zusammensetzungen mit verbessertem Lichtschutzfaktor, enthaltend mindestens ein Alkyl- und/oder Alkoxy-substituiertes Dibenzoylmethanderivat, Polysilicone-15, mindestens einen wasserlöslichen UV-Filter, umfassend ein Derivat der Benzimidazolsulfonsäure, sowie 0,4 - 15 Gew.-% einer Mischung aus mindestens einem nicht-alkoxylierten Ölsäureester mindestens eines linearen, gesättigten C₁₀-C₂₄-Alkanols und mindestens einem nicht-alkoxylierten Sorbitan-Ölsäure-Ester.

## Beschreibung

Die vorliegende Erfindung betrifft kosmetische und dermatologische Zusammensetzungen zum Schutz von Haut und Haar gegen UV-Strahlung.
Die hautschädigende Wirkung des ultravioletten Teils der Sonnenstrahlung ist bekannt. Die UV-Strahlung im Bereich zwischen 290 nm und 320 nm, dem so genannten UV-B-Bereich, kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben.
Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut, weitaus gefährlicher als UV-B-Strahlung ist. Auch kann der schädigende Einfluss der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.
Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.
Es ist daher von grundsätzlicher Wichtigkeit, dass kosmetische und dermatologische Lichtschutzzusammensetzungen sowohl gegen UV-B- als auch gegen UV-A-Strahlung ausreichenden Schutz bieten. Hierzu sind im Stand der Technik schon eine Vielzahl von kosmetischen Zusammensetzungen entwickelt worden, die mindestens eine UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UV-A-Filter und UV-B-Filter, je nachdem, in welchem Wellenlängenbereich das Absorptionsmaximum des Filters liegt.

### Aufgabe

Die Zusammensetzungen des Standes der Technik sind weiter verbesserbar. Insbesondere weisen Zusammensetzungen, die Kombinationen von UV-Filtersubstanzen oder anderen Wirkstoffen enthalten, bisweilen in Bezug auf die Einsatzkonzentrationen formulierungstechnische Schwierigkeiten auf, die das Erreichen guter UV-Schutzleistung erschweren. Ein weiterer Nachteil des Standes der Technik ist, dass übliche Lichtschutzformulierungen einen meist klebrigen Film auf der Haut hinterlassen. Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig verbesserte sensorische Eigenschaften aufweisen. Eine besondere Aufgabe bestand weiterhin darin, die UV-Schutzleistung bekannter und gesetzlich zugelassener UV-Filtersubstanzen durch spezifisch ausgewählte Rezepturformulierungen weiter zu steigern. Die erfindungsgemäßen Zusammensetzungen sollten ferner gute Haut pflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen.

### Stand der Technik

WO 2003/032943 A1 offenbart ein Emulgatorgemisch aus Cetearylolivat und Sorbitanolivat sowie eine Sonnenschutzemulsion, die dieses Emulgatorgemisch sowie 0,5 Gew.-% Benzophenon-3, 5 Gew.-% Ethylhexylmethoxycinnamat und 2 Gew.-% Titandioxid enthält und einen berechneten Lichtschutzfaktor von 8 aufweist. Diese Lichtschutzleistung ist unbefriedigend. WO 2003/032943 A1 enthält keine Hinweise darauf, dass sich mit Cetearylolivat und Sorbitanolivat der Lichtschutzfaktor erfindungsgemäß steigern lässt.

Gegenstand der vorliegenden Anmeldung sind kosmetische Zusammensetzungen in Form einer öl- und wasserhaltigen Dispersion, enthaltend mindestens einen öllöslichen UV-Filter, ausgewählt aus Alkyl- und/oder Alkoxy-substituierten Dibenzoylmethanderivaten, weiterhin Polysilicone-15 und mindestens einen wasserlöslichen UV-Filter, ausgewählt aus Derivaten der Benzimidazolsulfonsäure, weiterhin mindestens einen nicht-alkoxylierten Ölsäureester mindestens eines linearen, gesättigten C₁₀-C₂₄-Alkanols und mindestens einen nicht-alkoxylierten Sorbitan-Ölsäure-Ester, wobei die Gesamtmenge der Ester d) und e) 0,4 - 15 Gew.-%, bevorzugt 1 - 10 Gew.-%, besonders bevorzugt 2 - 8 Gew.-%, außerordentlich bevorzugt 3 - 5 Gew.-%, jeweils bezogen auf das Gesamtgewicht der Zusammensetzung, beträgt.
Gegenstand der Erfindung ist ferner die Verwendung der erfindungsgemäßen vorstehend definierten Zusammensetzungen zum Schutz von Haut und Haar gegen die schädlichen Wirkungen von UV-Strahlung, insbesondere UV-A-und UV-B-Strahlung, insbesondere Sonnenstrahlung. Bevorzugte Ausführungsformen der vorliegenden Erfindung ergeben sich aus den abhängigen Patentansprüchen.
Die erfindungsgemäßen Zusammensetzungen stellen in jeglicher Hinsicht überaus befriedigende Präparate dar, die im Hinblick auf die weiteren Bestandteile nicht auf eine spezielle Auswahl dieser weiteren Bestandteile begrenzt sind. Dementsprechend eignen sie sich ganz besonders, um als Grundlage für Zusammensetzungsformen mit vielfältigen Anwendungszwecken zu dienen. Die erfindungsgemäßen Zusammensetzungen zeigen überraschenderweise synergistisch verbesserte sensorische und kosmetische Eigenschaften, wie beispielsweise die gleichmäßige Verteilbarkeit auf der Haut oder das Einzugsvermögen in die Haut, und zeichnen sich gleichzeitig durch eine hohe Lichtschutzeffektivität bei gleichzeitig hervorragenden Hautpflegeeigenschaften aus.
Ein Maß für die UV-Schutzleistung stellt im Sinne der vorliegenden Erfindung beispielsweise der Lichtschutzfaktor (LSF bzw. SPF) dar.
Die kosmetischen und dermatologischen Zusammensetzungen im Sinne der vorliegenden Erfindung hinterlassen auf der Haut keinen schmierigen oder klebrigen Eindruck und sind ausgezeichnet hautverträglich.

### a) Alkyl- und/oder Alkoxy-substituiertes Dibenzoylmethanderivat

Erfindungsgemäß bevorzugte Alkyl- und/oder Alkoxy-substituierte Dibenzoylmethanderivate sind **dadurch gekennzeichnet, dass** einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxy, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als PARSOL^{®} 1789 von DSM oder als Eusolex^{®} 9020 von Merck KGaA erhältlich ist.

### b) Polysilicone-15

Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.

### c) Derivat der Benzimidazolsulfonsäure

Bevorzugte UV-Filtersubstanz(en) der Komponente (c) der erfindungsgemäßen Zusammensetzungen ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze, insbesondere das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das z. B. unter der Handelsbezeichnung Neo Heliopan AP von Symrise erhältlich ist. Weitere bevorzugte UV-Filtersubstanz(en) der Komponente (c) der erfindungsgemäßen Zusammensetzungen ist bzw. sind die 2-Phenylbenzimidazol-5-sulfonsäure (UV-B) und ihre Salze, insbesondere die Alkali-, Erdalkali-, Ammonium-, Alkylammonium-, Alkanolamin- und Glucammoniumsalze, bevorzugt die entsprechenden Natrium-, Kalium-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris(hydroxymethyl)aminomethan-Salze, insbesondere aber die 2-Phenylbenzimidazol-5-sulfonsäure selbst mit der INCI-Bezeichnung "Phenylbenzimidazole sulfonic acid" (CAS.-Nr. 27503-81-7), die z. B. unter den Handelsnamen Neo Heliopan Hydro von Symrise oder Eusolex 232 von Merck KGaA erhältlich ist.
In einer erfindungsgemäß besonders bevorzugten Ausführungsform sind die Salze der 2-Phenylbenzimidazol-5-sulfonsäure ausgewählt aus den Salzen dieser Säure mit basischen Aminosäuren, insbesondere mit Lysin, Arginin und/oder Histidin, wobei Arginin besonders bevorzugt ist. In einer weiteren erfindungsgemäß besonders bevorzugten Ausführungsform sind die Salze der Phenylen-1,4-bis-(2-benzimidazyl)-3,3'-5,5'-tetrasulfonsäure ausgewählt aus den Salzen dieser Säure mit basischen Aminosäuren, insbesondere mit Ornithin, Lysin, Arginin und/oder Histidin, wobei Arginin besonders bevorzugt ist.
Erfindungsgemäß besonders bevorzugte Lichtschutzfilter-Kombinationen (a), (b) und (c) umfassen 4-tert-Butyl-4'-Methoxydibenzoylmethan, Polysilicone-15 und 2-Phenylbenzimidazol-5-sulfonsäure. Weitere erfindungsgemäß besonders bevorzugte Lichtschutzfilter-Kombinationen (a), (b) und (c) umfassen 4-tert-Butyl-4'-Methoxydibenzoylmethan, Polysilicone-15 und Dinatriumphenylbenzimidazoltetrasulfonat.

Weitere erfindungsgemäß besonders bevorzugte Lichtschutzfilter-Kombinationen (a), (b) und (c) umfassen 4-tert-Butyl-4'-Methoxydibenzoylmethan, Polysilicone-15 und ein 2-Phenylbenzimidazol-5-sulfonsäure-Arginin-Salz.
Die oben genannten UV-Filtersubstanzen der Komponente (a) sind bevorzugt in einer Gesamtmenge von 0,5 Gew.-% bis 20 Gew.-%, besonders bevorzugt 1 bis 15 Gew.-%, insbesondere 2 bis 10 Gew.-% und außerordentlich bevorzugt 3 - 5 Gew.-%, enthalten, jeweils bezogen auf das Gesamtgewicht der erfindungsgemäßen Zusammensetzungen.
Polysilicone-15, die oben genannte Komponente (b), ist bevorzugt in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.
Die oben genannten UV-Filtersubstanzen der Komponente (c) sind bevorzugt in einer Gesamtmenge von 0,5 Gew.-% bis 15 Gew.-%, besonders bevorzugt 1 bis 10 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.
Bevorzugte erfindungsgemäße Zusammensetzungen sind **dadurch gekennzeichnet, dass** die UV-Filter a), b) und c) in einem Gewichtsverhältnis zueinander von a) : b) : c) wie (0,8 - 1,5) : (0,8 - 1,5) : (0,8 - 1,5), bevorzugt wie (0,9 - 1,2) : (0,9 - 1,2) : (0,9 - 1,2), besonders bevorzugt wie (1 - 1,1) : (1 - 1,1) : (1 - 1,1), enthalten sind.
Um den Lichtschutzfaktor weiter zu erhöhen, können die erfindungsgemäßen Zusammensetzungen bevorzugt mindestens eine weitere organische und/oder anorganische UV-Filtersubstanz enthalten. Weitere bevorzugte UV-Filtersubstanzen, die neben denen der Komponenten (a), (b) und (c) in den erfindungsgemäßen Zusammensetzungen eingesetzt werden, sind im Folgenden genannt.

### Triazinderivate

### UV-Filtersubstanzen auf Basis von Triazinderivaten, die das nachfolgende Strukturmotiv

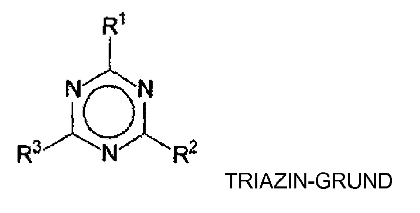

aufweisen, sind im Stand der Technik bekannt, beispielsweise aus EP 775698, EP 878469 und EP 1027881.
Hinsichtlich der C₃-Achse des Triazin-Grundkörpers dieser Verbindungen sind sowohl symmetrische Substitution als auch unsymmetrische Substitution denkbar.
In diesem Sinne symmetrisch substituierte s-Triazine weisen drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt.
Hinsichtlich der C₃-Achse des Triazin-Grundkörpers symmetrische Triazinderivate sind bevorzugt solche der allgemeinen Formel TRIAZIN-GRUND mit R¹ = R² = R³ = -NH-Phe-COOR, mit Phe = Phenyl-Rest, bei dem die Substituenten -NH und -COOR in para-Position zueinander stehen.
Besonders bevorzugte derartige symmetrische Traizinderivate sind 4,4',4"-(1,3,5-Triazin-2,4,6-triyltriimino)-tris-benzoesäure-tris(alkylester). Ein besonders bevorzugter derartiger Ester ist 4,4',4"-(1,3,5-Triazin-2,4,6-triyltriimino)-tris-benzoesäure-tris(2-ethylhexylester) [INCI: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist. Erfindungsgemäß bevorzugte unsymmetrische Triazinderivate sind beispielweise solche, die in EP 775698 offenbart sind : 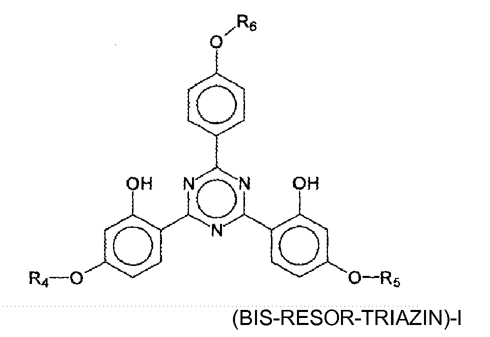 und/oder

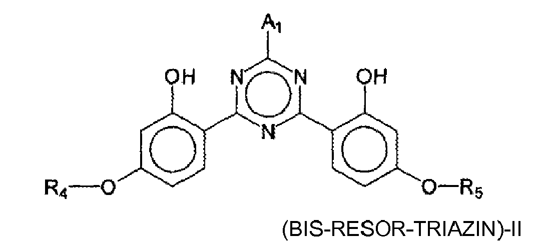

Alle in EP 775698 erwähten so gennanten Bis-Resorcinyltriazine, seien sie durch generische oder durch konkrete Formeln offenbart, sind vorteilhaft im Sinne der vorliegenden Erfindung.
Ganz besonders bevorzugt, werden R₄ und R₅ aus der Gruppe der verweigten und unverzweigten Alkylgruppen von 1 bis 18 Kohlenstoffatomen gewählt. Auch können die Alkylgruppen wiederum vorteilhaft mit Silyloxygruppen substituiert sein.
A₁ stellt bevorzugt einen substituiierten homo- oder heterocyclyschen aromatischen Fünfring oder Sechsring dar.
Ganz besonders bevorzugte optionale UV-Filtersubstanzen sind ausgewählt aus unsymmetrich substituierten s-Triazin-Verbindungen der allgemeinen Formel (BIS-RESOR-TRIAZIN)-I, in der R₆ ein Wasserstoffatom oder eine verzweigte oder unverzweigte Alkylgruppe mit 1 bis 10 Kohlenstoffatomen darstellt. Eine besonders bevorzugte Verbindung der allgemeinen Formel (BIS-RESOR-TRIAZIN)-1, in der R₄ und R₅ jeweils eine 2-Ethylhexyl-Gruppe und R₆ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.
Eine weitere, erfindungsgemäß bevorzugte optionale unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit R¹ = R² -NH-Phe-COOR, mit Phe = Phenyl-Rest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein effektiver UV-A-Filter, ist unter der Handelsbezeichnung UVASORB HEB bei Sigma 3V erhältlich ist.
Weitere, erfindungsgemäß bevorzugte optionale UV-Filtersubstanzen auf Basis von s-TriazinVerbindungen sind:
- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenyl-amino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3 ,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.

Die s-Triazinderivate werden bevorzugt in die Ölphase der erfindungsgemäßen kosmetischen oder dermatologischen Zusammensetzungen eingearbeitet.

### Benzotriazole

Eine weitere bevorzugte optionale UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel 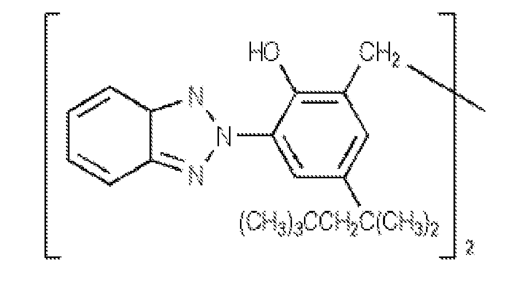 , unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert.
Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.
Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.
Weitere bevorzugte UV-Filtersubstanzen, die neben denen der Komponenten (a), (b) und (c) in den erfindungsgemäßen Zusammensetzungen eingesetzt werden, sind im Folgenden genannt:
- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor], das von Merck unter dem Namen Eusolex 6300 vertrieben wird;
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)-sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich).
- 4-Aminobenzoesäure-Derivate, bevorzugt 4-(Dimethylamino)-benzoesäure(2-ethylhexyl)-ester, 4-(Dimethylamino)benzoesäureamylester;
- 2-Aminobenzoësäure-Derivate
- Ester der Zimtsäure, bevorzugt 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethyl-hexylester (= Ethylhexyl-2-cyano-3,3-diphenylacrylat (Octocrylene)),
- Ester der Salicylsäure, bevorzugt Salicylsäure(2-ethylhexyl)ester (2-Ethylhexylsalicylat (= Octylsalicylat)), Salicylsäure(4-isopropylbenzyl)ester (4-Isopropylbenzylsalicylat), Salicylsäurehomomenthylester (Homomenthylsalicylat, Homosalate).
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus bei der Firma BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)ester,
- sowie an Polymere gebundene UV-Filter, die von Komponente (b) verschieden sind.
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine).
   Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4`methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Die Liste der genannten optionalen UV-Filtersubstanzen, die im Sinne der vorliegenden Erfindung eingesetzt werden können, soll selbstverständlich nicht limitierend sein.
Die Gesamtmenge an der mindestens einen optionalen organischen UV-Filtersubstanz in den erfindungsgemäßen kosmetischen oder dermatologischen Zusammensetzungen beträgt bevorzugt 0,5 - 20 Gew.-%, besonders bevorzugt 1 - 15 Gew.-%, außerordentlich bevorzugt 2 - 10 Gew.-%, jeweils bezogen auf das Gesamtgewicht der erfindungsgemäßen Zusammensetzung.
Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira.
Erfindungsgemäß bevorzugt ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.
Die erfindungsgemäßen Zusammensetzungen liegen in Form einer öl- und wasserhaltigen Dispersion vor. Bevorzugt liegen die erfindungsgemäßen Zusammensetzungen in Form einer öl- und wasserhaltigen Emulsion vor. Besonders bevorzugt liegen die erfindungsgemäßen Zusammensetzungen in Form einer Öl-in-Wasser-Emulsion vor.
Die erfindungsgemäßen Zusammensetzungen sind **dadurch gekennzeichnet, dass** sie mindestens einen nicht-alkoxylierten Ölsäureester mindestens eines linearen, gesättigten C₁₀-C₂₄-Alkanols und mindestens einen nicht-alkoxylierten Sorbitan-Ölsäure-Ester enthalten, wobei die Gesamtmenge dieser Ester 0,5 - 15 Gew.-%, bezogen auf das Gesamtgewicht der Zusammensetzung, beträgt.

Die Moleküle des mindestens einen nicht-alkoxylierten Ölsäureesters mindestens eines linearen, gesättigten C₁₀-C₂₄-Alkanols bestehen nur aus der Ölsäure-Gruppe und dem hiermit veresterten Alkanolrest, ausgewählt aus C₁₀-C₂₄-Alkanol-Resten. Der Ester enthält also nicht zusätzlich noch Alkylenoxid-Einheiten oder andere Molekülbausteine.
Die Moleküle des mindestens einen nicht-alkoxylierten Sorbitan-Ölsäure-Esters bestehen nur aus Säureresten aus der Ölsäure-Gruppe und den hiermit veresterten Sorbitan-Resten. Der Ester enthält also nicht zusätzlich noch Alkylenoxid-Einheiten oder andere Molekülbausteine.

Bevorzugt werden die vorgenannten Ester als vorgemischtes Compound eingesetzt, um die erfindungsgemäßen Zusammensetzungen als Emulgator zu stabilisieren.

Der mindestens eine lineare, gesättigte C₁₀-C₂₄-Alkanol ist bevorzugt ausgewählt aus Caprinalkohol, n-Undecylalkohol, Laurylalkohol, Tridecylalkohol, Myristylalkohol, n-Pentadecylalkohol, Cetylalkohol, n-Heptadecylalkohol, Stearylalkohol, n-Nonadecylalkohol, Arachylalkohol, Heneicosylalkohol, Behenylalkohol und Lignocerylalkohol sowie deren technischen Mischungen. Besonders bevorzugt sind Myristylalkohol, Cetylalkohol, Stearylalkohol und Arachylalkohol sowie deren technischen Mischungen. Außerordentlich bevorzugt sind Cetylalkohol und Stearylalkohol sowie deren technischen Mischungen, die auch als Cetearylalkohol bezeichnet werden.
Bevorzugte kosmetische Zusammensetzungen sind daher **dadurch gekennzeichnet, dass** der Ester d) ausgewählt ist aus Cetyloleat, Stearyloleat und Cetearyloleat.

Besonders bevorzugt ist es, wenn die Ölsäure, aus denen die beiden erfindungsgemäß eingesetzten Ölsäureester d) und e) hergestellt sind, nicht in reiner Form, sondern in einer Fettsäuremischung vorliegt, wie sie bei der Dampfspaltung von natürlichen Ölen und Fetten in Fettsäuren und Glycerin ohne quantitative Veränderung der Fettsäurefraktionen erhalten wird. Diese Fettsäuremischungen enthalten neben Ölsäure vor allem auch Palmitinsäure und Linolsäure. Besonders bevorzugte kosmetische Zusammensetzungen sind daher **dadurch gekennzeichnet, dass** als Ester d) ein Gemisch aus mindestens einem Ölsäureester mindestens eines linearen, gesättigten C₁₀-C₂₄-Alkanols, mindestens einem Palmitinsäureester mindestens eines linearen, gesättigten C₁₀-C₂₄-Alkanols und mindestens einem Linolsäureester mindestens eines linearen, gesättigten C₁₀-C₂₄-Alkanols enthalten ist.
Weitere besonders bevorzugte kosmetische Zusammensetzungen sind **dadurch gekennzeichnet, dass** als Ester e) ein Gemisch aus mindestens einem nicht-alkoxylierten Sorbitan-Ölsäure-Ester, mindestens einem nicht-alkoxylierten Sorbitan-Palmitinsäure-Ester und mindestens einem nicht-alkoxylierten Sorbitan-Linolsäure-Ester enthalten ist.
Die bei der Herstellung der erfindungsgemäß eingesetzten Ester d) und e) eingesetzten Fettsäuremischungen aus natürlichen Ölen und Fetten stammen bevorzugt aus natürlichen Ölen und Fetten, die ausgewählt sind aus Olivenöl, High oleic-Sonnenblumenöl, High oleic-Distelöl, Erdnussöl, Haselnussöl, Macadamianussöl, Pekannussöl, Arganöl, Avocadoöl, LEAR-Rapsöl, Mandelöl, Aprikosenkernöl, Sesamöl, Kürbiskernöl, Maiskeimöl, Gänsefett, Hammeltalg, Rindertalg und Schweineschmalz. Ebenfalls geeignet sind die Fettsäuremischungen aus natürlichen Ölen und Fetten, die ausgewählt sind aus Distelöl, Sonnenblumenöl, Sojaöl, Leinöl (Flachsöl), Baumwollsaatöl, Weizenkeimöl, Koriandersamenöl, Mohnöl, Pfirsichkernöl, und Nachtkerzenöl.
Die typische Fettsäurezusammensetzung natürlicher Öle und Fette ist in der folgenden Tabelle dargestellt (Mengenangaben in Gew.-%, bezogen auf Gesamtfettsäuren):

**Tabelle 1: Fettsäure-Zusammensetzung wichtiger pflanzlicher Fette und Öle (Anzahl Kohlenstoff-Atome : Anzahl Doppelbindungen).**

| | Olivenöl | Mandelöl | Erdnussöl | Haselnussöl | LEAR-Rapsöl | Gänsefett | Maiskeimöl | High oleic-Sonnenblumenöl | High oleic-Distelöl |
|---|---|---|---|---|---|---|---|---|---|
| C14:0 | ≤ 0,05 | NN | ≤ 0,1 | ≤ 0,1 | ≤ 0,2 | NN | ≤ 0,3 | ≤ 0,1 | ≤ 0,2 |
| C16:0 | 7,5-20 | 6-8 | 8-14 | 5-9 | 2,5-7 | 22-25 | 8,6-16,5 | 2,6-5 | 3,6-6 |
| C16:1 | 0,3-3,5 | ≤ 1 | ≤ 0,2 | ≤ 0,3 | ≤ 0,6 | 3-3,7 | ≤ 0,5 | ≤ 0,1 | ≤ 0,2 |
| C17:0 | ≤ 0,3 | NN | ≤ 0,1 | ≤ 0,1 | ≤ 0,3 | ≤ 0,2 | ≤ 0,1 | ≤ 0,1 | ≤ 0,1 |
| C17:1 | ≤ 0,3 | NN | ≤ 0,1 | NN | ≤ 0,3 | ≤ 0,2 | ≤ 0,1 | ≤ 0,1 | ≤ 0,1 |
| C18:0 | 0,5-5 | 1-2 | 1-4,5 | 1-4 | 0,8-3 | 6,5-9,5 | ≤ 3,3 | 2,9-6,2 | 1,5-2,4 |
| C18:1 | 55-83 | 64-82 | 35-69 | 66-83 | 51-70 | 51-57 | 20-42,2 | 75-90,7 | 70-83,7 |
| C18:2 | 3,5-21 | 8-28 | 12-43 | 8-25 | 15-30 | 9,1-10 | 34-65,6 | 2,1-17 | 9-19,9 |
| C18:3 | ≤ 1 | ≤ 0,2 | ≤ 0,3 | ≤ 0,6 | 5-14 | 0,3-0,5 | ≤ 2 | ≤ 0,3 | ≤ 1,2 |
| C20:0 | ≤ 0,6 | ≤ 0,1 | 1-2 | ≤ 0,3 | 0,2-1,2 | NN | 0,3-1 | 0,2-0,5 | 0,3-0,6 |
| C20:1 | ≤ 0,4 | ≤ 0,1 | 0,7-1,7 | NN | 0,1-4,3 | NN | 0,2-0,6 | 0,1-0,5 | 0,1-0,5 |
| C22:0 | ≤ 0,2 | ≤ 0,2 | 1,5-4,5 | NN | ≤ 0,6 | NN | ≤ 0,5 | 0,5-1,6 | ≤ 1,0 |
| C24:0 | ≤ 0,2 | NN | 0,5-2,5 | NN | ≤ 0,3 | NN | ≤ 0,5 | ≤ 0,5 | ≤ 0,2 |
| C14:0 | Myristinsäure | | | | | | | | |
| C16:0 | Palmitinsäure | | | | | | | | |
| C16:1 | Palmitoleinsäure | | | | | | | | |
| C17:0 | Margarinsäure | | | | | | | | |
| C17:1 | Δ9cis-Heptadecensäure | | | | | | | | |
| C18:0 | Stearinsäure | | | | | | | | |
| C18:1 | Ölsäure | | | | | | | | |
| C18:2 | Linolsäure | | | | | | | | |
| C18:3 | Linolensäure | | | | | | | | |
| C18:4 | Arachidonsäure | | | | | | | | |
| C20:1 | Gadoleinsäure | | | | | | | | |
| C22:0 | Behensäure | | | | | | | | |
| C24:0 | Lignocerinsäure | | | | | | | | |

Fettsäuremischungen aus Olivenöl sind sowohl für die Ester mit mindestens einem linearen, gesättigten C₁₀-C₂₄-Alkanol als auch mit Sorbitan erfindungsgemäß besonders bevorzugt.
Ein außerordentlich bevorzugter Ester d) ist Cetearylolivat. Ein außerordentlich bevorzugter Ester e) stammt aus Sorbitanolivat. Weiterhin sind als Mischungen d) plus e) Mischungen aus Cetearylolivat und Sorbitanolivat außerordentlich bevorzugt.
Wie die beigefügten Vergleichsversuche zeigen, führt der Einsatz der Ölsäureester-Mischungen, insbesondere der Olivenölfettsäureester-Mischungen, zu einer Verdoppelung des in vivo gemessenen Lichtschutzfaktors gegenüber einer Rezeptur, die sich nur durch den Emulgator von der erfindungsgemäßen Rezeptur unterscheidet.
Die Gesamtmenge der Ester d) und e) beträgt 0,4 - 15 Gew.-%, bevorzugt 1 - 10 Gew.-%, bevorzugt 2 - 8 Gew.-%, besonders bevorzugt 3 - 5 Gew.-%, jeweils bezogen auf das Gesamtgewicht der erfindungsgemäßen Zusammensetzung.
In einer bevorzugten Ausführungsform der Erfindung ist die Mischung von Cetearylolivat und Sorbitanolivat in einer Gesamtmenge von 0,5 - 15 Gew.-%, bevorzugt 1 - 10 Gew.-%, bevorzugt 2 - 8 Gew.-%, besonders bevorzugt 3 - 5 Gew.-%, jeweils bezogen auf das Gesamtgewicht der erfindungsgemäßen Zusammensetzung, enthalten.
Das Gewichtsverhältnis des Esters/der Ester d) zu dem Ester/den Estern e) liegt bevorzugt im Bereich von 20:80 bis 80:20, bevorzugt im Bereich von 30:70 bis 70:30, besonders bevorzugt im Bereich von 40:60 bis 50:50.
In einer besonders bevorzugten Ausführungsform der Erfindung sind Cetearyloleat und Sorbitanoleat in einem Gewichtsverhältnis von Cetearyloleat zu Sorbitanoleat im Bereich von 20:80 bis 80:20, bevorzugt im Bereich von 30:70 bis 70:30, besonders bevorzugt im Bereich von 40:60 bis 50:50, enthalten. Außerordentlich bevorzugt ist ein Gewichtsverhältnis von Cetearyloleat zu Sorbitanoleat im Bereich von 40:60 bis 70:30.
In einer anderen besonders bevorzugten Ausführungsform der Erfindung sind Cetyloleat und Sorbitanoleat in einem Gewichtsverhältnis von Cetyloleat zu Sorbitanoleat im Bereich von 20:80 bis 80:20, bevorzugt im Bereich von 30:70 bis 70:30, besonders bevorzugt im Bereich von 40:60 bis 50:50, enthalten. Außerordentlich bevorzugt ist ein Gewichtsverhältnis von Cetyloleat zu Sorbitanoleat im Bereich von 40:60 bis 70:30.
In einer weiteren besonders bevorzugten Ausführungsform der Erfindung sind Cetearylolivat und Sorbitanolivat in einem Gewichtsverhältnis von Cetearylolivat zu Sorbitanolivat im Bereich von 20:80 bis 80:20, bevorzugt im Bereich von 30:70 bis 70:30, besonders bevorzugt im Bereich von 40:60 bis 50:50, enthalten. Außerordentlich bevorzugt ist ein Gewichtsverhältnis von Cetearylolivat zu Sorbitanolivat im Bereich von 40:60 bis 70:30.
In einer besonders bevorzugten Ausführungsform der Erfindung ist die Mischung von Cetearylolivat und Sorbitanolivat in einer Gesamtmenge von 0,5 - 15 Gew.-%, bevorzugt 1 - 10 Gew.-%, bevorzugt 2 - 8 Gew.-%, besonders bevorzugt 3 - 5 Gew.-%, jeweils bezogen auf das Gesamtgewicht der erfindungsgemäßen Zusammensetzung, enthalten, wobei das Gewichtsverhältnis von Cetearylolivat zu Sorbitanolivat im Bereich von 20:80 bis 80:20, bevorzugt im Bereich von 30:70 bis 70:30, besonders bevorzugt im Bereich von 40:60 bis 50:50 liegt.
Der mindestens eine nicht-alkoxylierte Ölsäureester mindestens eines linearen, gesättigten C₁₀-C₂₄-Alkanols ist bevorzugt in einer Gesamtmenge von 0,1 - 12 Gew.-%, weiter bevorzugt 1 - 8 Gew.-%, besonders bevorzugt 2 - 5 Gew.-%, außerordentlich bevorzugt 2,5 - 3,5 Gew.-%, enthalten, jeweils bezogen auf das Gesamtgewicht der erfindungsgemäßen Zusammensetzung.
Der mindestens eine nicht-alkoxylierte Sorbitan-Ölsäure-Ester ist bevorzugt in einer Gesamtmenge von 0,1 - 12 Gew.-%, weiter bevorzugt 0,5 - 5 Gew.-%, besonders bevorzugt 1 - 4 Gew.-%, außerordentlich bevorzugt 1,5 - 2,5 Gew.-%, enthalten, jeweils bezogen auf das Gesamtgewicht der erfindungsgemäßen Zusammensetzung.

Erfindungsgemäß besonders bevorzugte Zusammensetzungen enthalten 4-tert-Butyl-4'-Methoxydibenzoylmethan, Polysilicone-15 und 2-Phenylbenzimidazol-5-sulfonsäure sowie 0,4 - 15 Gew.-% einer Mischung aus Cetearyloleat und Sorbitanoleat.
Weitere erfindungsgemäß besonders bevorzugte Zusammensetzungen enthalten 4-tert-Butyl-4'-Methoxydibenzoylmethan, Polysilicone-15 und Dinatriumphenylbenzimidazoltetrasulfonat sowie 0,5 - 15 Gew.-% einer Mischung aus Cetearylolivat und Sorbitanolivat.
Weitere erfindungsgemäß besonders bevorzugte Zusammensetzungen enthalten 4-tert-Butyl-4'-Methoxydibenzoylmethan, Polysilicone-15 und ein 2-Phenylbenzimidazol-5-sulfonsäure-Arginin-Salz sowie 0,5 - 15 Gew.-% einer Mischung aus Cetearylolivat und Sorbitanolivat.
Weitere erfindungsgemäß besonders bevorzugte Zusammensetzungen enthalten 0,5 Gew.-% bis 20 Gew.-% 4-tert-Butyl-4'-Methoxydibenzoylmethan, 0,5 Gew.-% bis 10 Gew.-% Polysilicone-15 und 0,5 Gew.-% bis 15 Gew.-% 2-Phenylbenzimidazol-5-sulfonsäure sowie 0,5 - 15 Gew.-% einer Mischung aus Cetearylolivat und Sorbitanolivat.
Weitere erfindungsgemäß besonders bevorzugte Zusammensetzungen enthalten 0,5 Gew.-% bis 20 Gew.-% 4-tert-Butyl-4'-Methoxydibenzoylmethan, 0,5 Gew.-% bis 10 Gew.-% Polysilicone-15 und 0,5 Gew.-% bis 15 Gew.-% Dinatriumphenylbenzimidazoltetrasulfonat sowie 0,5 - 15 Gew.-% einer Mischung aus Cetearylolivat und Sorbitanolivat.
Weitere erfindungsgemäß besonders bevorzugte Zusammensetzungen enthalten 0,5 Gew.-% bis 20 Gew.-% 4-tert-Butyl-4'-Methoxydibenzoylmethan, 0,5 Gew.-% bis 10 Gew.-% Polysilicone-15 und 0,5 Gew.-% bis 15 Gew.-% eines 2-Phenylbenzimidazol-5-sulfonsäure-Arginin-Salzes sowie 0,5 - 15 Gew.-% einer Mischung aus Cetearylolivat und Sorbitanolivat.
Weitere erfindungsgemäß besonders bevorzugte Zusammensetzungen enthalten 1 bis 15 Gew.-% 4-tert-Butyl-4'-Methoxydibenzoylmethan, 1 - 7 Gew.-% Polysilicone-15 und 1 - 10 Gew.-% 2-Phenylbenzimidazol-5-sulfonsäure sowie 1 - 10 Gew.-% einer Mischung aus Cetearylolivat und Sorbitanolivat.
Weitere erfindungsgemäß besonders bevorzugte Zusammensetzungen enthalten 1 bis 15 Gew.-% 4-tert-Butyl-4'-Methoxydibenzoylmethan, 1 - 7 Gew.-% Polysilicone-15 und 1 - 10 Gew.-% Dinatriumphenylbenzimidazoltetrasulfonat sowie 1 - 10 Gew.-% einer Mischung aus Cetearylolivat und Sorbitanolivat.
Weitere erfindungsgemäß besonders bevorzugte Zusammensetzungen enthalten 1 bis 15 Gew.-% 4-tert-Butyl-4'-Methoxydibenzoylmethan, 1 - 7 Gew.-% Polysilicone-15 und 1 - 10 Gew.-% eines 2-Phenylbenzimidazol-5-sulfonsäure-Arginin-Salzes sowie 1 - 10 Gew.-% einer Mischung aus Cetearylolivat und Sorbitanolivat.
Weitere erfindungsgemäß besonders bevorzugte Zusammensetzungen enthalten 2 - 10 Gew.-% 4-tert-Butyl-4'-Methoxydibenzoylmethan, 2 - 5 Gew.-% Polysilicone-15 und 2 - 5 Gew.-% 2-Phenylbenzimidazol-5-sulfonsäure sowie 2 - 8 Gew.-% einer Mischung aus Cetearylolivat und Sorbitanolivat.
Weitere erfindungsgemäß besonders bevorzugte Zusammensetzungen enthalten 2 - 10 Gew.-% 4-tert-Butyl-4'-Methoxydibenzoylmethan, 2 - 5 Gew.-% Polysilicone-15 und 2 - 5 Gew.-% Dinatriumphenylbenzimidazoltetrasulfonat sowie 2 - 8 Gew.-% einer Mischung aus Cetearylolivat und Sorbitanolivat.
Weitere erfindungsgemäß besonders bevorzugte Zusammensetzungen enthalten 2 - 10 Gew.-% 4-tert-Butyl-4'-Methoxydibenzoylmethan, 2 - 5 Gew.-% Polysilicone-15 und 2 - 5 Gew.-% eines 2-Phenylbenzimidazol-5-sulfonsäure-Arginin-Salzes sowie 2 - 8 Gew.-% einer Mischung aus Cetearylolivat und Sorbitanolivat.
Weitere erfindungsgemäß besonders bevorzugte Zusammensetzungen enthalten 4-tert-Butyl-4'-Methoxydibenzoylmethan, Polysilicone-15 und Dinatriumphenylbenzimidazoltetrasulfonat sowie 0,4 - 15 Gew.-% einer Mischung aus Cetearyloleat und Sorbitanoleat.

Weitere erfindungsgemäß besonders bevorzugte Zusammensetzungen enthalten 4-tert-Butyl-4'-Methoxydibenzoylmethan, Polysilicone-15 und ein 2-Phenylbenzimidazol-5-sulfonsäure-Arginin-Salz sowie 0,4 - 15 Gew.-% einer Mischung aus Cetearyloleat und Sorbitanoleat.
Weitere erfindungsgemäß besonders bevorzugte Zusammensetzungen enthalten 0,5 Gew.-% bis 20 Gew.-% 4-tert-Butyl-4'-Methoxydibenzoylmethan, 0,5 Gew.-% bis 10 Gew.-% Polysilicone-15 und 0,5 Gew.-% bis 15 Gew.-% 2-Phenylbenzimidazol-5-sulfonsäure sowie 0,4 - 15 Gew.-% einer Mischung aus Cetearyloleat und Sorbitanoleat.
Weitere erfindungsgemäß besonders bevorzugte Zusammensetzungen enthalten 0,5 Gew.-% bis 20 Gew.-% 4-tert-Butyl-4'-Methoxydibenzoylmethan, 0,5 Gew.-% bis 10 Gew.-% Polysilicone-15 und 0,5 Gew.-% bis 15 Gew.-% Dinatriumphenylbenzimidazoltetrasulfonat sowie 0,4 - 15 Gew.-% einer Mischung aus Cetearyloleat und Sorbitanoleat.
Weitere erfindungsgemäß besonders bevorzugte Zusammensetzungen enthalten 0,5 Gew.-% bis 20 Gew.-% 4-tert-Butyl-4'-Methoxydibenzoylmethan, 0,5 Gew.-% bis 10 Gew.-% Polysilicone-15 und 0,5 Gew.-% bis 15 Gew.-% eines 2-Phenylbenzimidazol-5-sulfonsäure-Arginin-Salzes sowie 0,4 - 15 Gew.-% einer Mischung aus Cetearyloleat und Sorbitanoleat.
Weitere erfindungsgemäß besonders bevorzugte Zusammensetzungen enthalten 1 bis 15 Gew.-% 4-tert-Butyl-4'-Methoxydibenzoylmethan, 1 - 7 Gew.-% Polysilicone-15 und 1 - 10 Gew.-% 2-Phenylbenzimidazol-5-sulfonsäure sowie 1 - 10 Gew.-% einer Mischung aus Cetearyloleat und Sorbitanoleat.
Weitere erfindungsgemäß besonders bevorzugte Zusammensetzungen enthalten 1 bis 15 Gew.-% 4-tert-Butyl-4'-Methoxydibenzoylmethan, 1 - 7 Gew.-% Polysilicone-15 und 1 - 10 Gew.-% Dinatriumphenylbenzimidazoltetrasulfonat sowie 1 - 10 Gew.-% einer Mischung aus Cetearyloleat und Sorbitanoleat.
Weitere erfindungsgemäß besonders bevorzugte Zusammensetzungen enthalten 1 bis 15 Gew.-% 4-tert-Butyl-4'-Methoxydibenzoylmethan, 1 - 7 Gew.-% Polysilicone-15 und 1 - 10 Gew.-% eines 2-Phenylbenzimidazol-5-sulfonsäure-Arginin-Salzes sowie 1 - 10 Gew.-% einer Mischung aus Cetearyloleat und Sorbitanoleat.
Weitere erfindungsgemäß besonders bevorzugte Zusammensetzungen enthalten 2 - 10 Gew.-% 4-tert-Butyl-4'-Methoxydibenzoylmethan, 2 - 5 Gew.-% Polysilicone-15 und 2 - 5 Gew.-% 2-Phenylbenzimidazol-5-sulfonsäure sowie 2 - 8 Gew.-% einer Mischung aus Cetearyloleat und Sorbitanoleat.
Weitere erfindungsgemäß besonders bevorzugte Zusammensetzungen enthalten 2 - 10 Gew.-% 4-tert-Butyl-4'-Methoxydibenzoylmethan, 2 - 5 Gew.-% Polysilicone-15 und 2 - 5 Gew.-% Dinatriumphenylbenzimidazoltetrasulfonat sowie 2 - 8 Gew.-% einer Mischung aus Cetearyloleat und Sorbitanoleat.
Weitere erfindungsgemäß besonders bevorzugte Zusammensetzungen enthalten 2 - 10 Gew.-% 4-tert-Butyl-4'-Methoxydibenzoylmethan, 2 - 5 Gew.-% Polysilicone-15 und 2 - 5 Gew.-% eines 2-Phenylbenzimidazol-5-sulfonsäure-Arginin-Salzes sowie 2 - 8 Gew.-% einer Mischung aus Cetearyloleat und Sorbitanoleat.

Alle Mengenangaben in Gew.-% beziehen sich auf das Gesamtgewicht der erfindungsgemäßen Zusammensetzung, sofern nicht etwas definiert ist.
Zur weiteren Steigerung des Lichtschutzfaktors und/oder zur Verbesserung des Hautgefühls enthalten die erfindungsgemäßen kosmetischen und dermatologischen Zusammensetzungen optional mindestens einen Feststoff, der aus Siliciumdioxid-Partikeln, die nicht organisch oder anorganisch modifiziert sind und keine organische oder anorganische Beschichtung aufweisen, ausgewählt ist, in einer Gesamtmenge von 0,2 - 5 Gew.-%, bezogen auf die gesamte Zusammensetzung. Das Merkmal "keine organische oder anorganische Beschichtung", bezieht sich erfindungsgemäß auf den Zustand der Siliciumdioxid-Partikel, bevor sie mit den übrigen Bestandteilen der erfindungsgemäßen Zusammensetzungen vermischt werden. Nach dem Vermischen ist es durchaus möglich, dass einzelne Rezepturbestandteile an der Oberfläche der Siliciumdioxid-Partikel sorbiert sind; das Merkmal "keine organische oder anorganische Beschichtung" bleibt davon aber unbenommen. Auch möglicherweise sorbierte Gase gelten erfindungsgemäß nicht als Beschichtung.
Besonders bevorzugt sind Siliciumdioxid-Partikel enthalten, die durch Kieselsäure-Fällung erhältlich sind. Besonders bevorzugt sind Siliciumdioxid-Partikel enthalten, die zu mehr als 80 Gew.-% sphärische Partikel umfassen. Besonders bevorzugt sind Siliciumdioxid-Partikel enthalten, die einen zahlenmittleren Partikeldurchmesser im Bereich von 2 - 15 µm, besonders bevorzugt im Bereich von 5 - 10 µm, aufweisen. Besonders bevorzugt sind Siliciumdioxid-Partikel enthalten, die eine Ölabsorptionskapazität von 0,7 - 1,5 cm³ flüssiges Paraffin pro Gramm trockenes Siliciumdioxid, aufweisen, gemessen nach DIN 53601. Besonders bevorzugt sind Siliciumdioxid-Partikel enthalten, die durch Kieselsäure-Fällung erhältlich sind und zu mehr als 80 Gew.-% sphärische Partikel umfassen. Besonders bevorzugt sind Siliciumdioxid-Partikel enthalten, die durch Kieselsäure-Fällung erhältlich sind und einen zahlenmittleren Partikeldurchmesser im Bereich von 2 - 15 µm, besonders bevorzugt im Bereich von 5 - 10 µm, aufweisen. Besonders bevorzugt sind Siliciumdioxid-Partikel enthalten, die durch Kieselsäure-Fällung erhältlich sind, zu mehr als 80 Gew.-% sphärische Partikel umfassen und einen zahlenmittleren Partikeldurchmesser im Bereich von 2 - 15 µm, besonders bevorzugt im Bereich von 5 - 10 µm, aufweisen. Besonders bevorzugt sind Siliciumdioxid-Partikel enthalten, die durch Kieselsäure-Fällung erhältlich sind, zu mehr als 80 Gew.-% sphärische Partikel umfassen, einen zahlenmittleren Partikeldurchmesser im Bereich von 2 - 15 µm, besonders bevorzugt im Bereich von 5 - 10 µm, und eine Ölabsorptionskapazität von 0,7 - 1,5 cm³ flüssiges Paraffin pro Gramm trockenes Siliciumdioxid, gemessen nach DIN 53601, aufweisen. Besonders bevorzugte Siliciumdioxid-Partikel dieser Art sind als Handelsprodukt SB-705 der Firma Miyoshi Kasei, erhältlich, ein sphärisches Kieselgel mit der INCI-Bezeichnung Silica, das einen zahlenmittleren Teilchendurchmesser von 5 - 6 µm und eine spezifische Oberfläche von etwa 600 m²/g aufweist. Weitere bevorzugte Siliciumdioxid-Partikel sind als Handelsprodukt Aerosil von Evonik Degussa erhältlich: Aerosil 130, Aerosil 200, Aerosil 255, Aerosil 300 oder Aerosil 380. Weitere bevorzugte Siliciumdioxid-Partikel sind ebenfalls als Handelsprodukte erhältlich: CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), Cosmedia Silc (Cognis), Neosil PC 50 S (Ineos Silicas), Sorbosil BFG 54 (Ineos Silicas), Sorbosil AC33 (Ineos Silicas), Sorbosil AC 35 (Ineos Silicas), Sorbosil AC 37 (Ineos Silicas), Sorbosil AC 39 (Ineos Silicas), Wacker HDK H 30 (Wacker-Chemie), Wacker HDK N 20 (Wacker-Chemie), Wacker HDK S 13 (Wacker-Chemie), Wacker HDK T 30 (Wacker-Chemie), Wacker HDK V 15 (Wacker-Chemie).
Besonders bevorzugte erfindungsgemäße Zusammensetzungen enthalten Siliciumdioxid-Partikel mit den vorgenannten Eigenschaften in einer Gesamtmenge von 0,2 - 5 Gew.-%, bevorzugt 1 - 4 Gew.-%, besonders bevorzugt 1,5 - 3,5 Gew.-%, außerordentlich bevorzugt 2 - 3 Gew.-% und weiter bevorzugt 2,5 - 2,8 Gew.-%, jeweils bezogen auf die gesamte Zusammensetzung.
Um die haptischen Eigenschaften und/oder den Lichtschutzfaktor der erfindungsgemäßen Zusammensetzungen weiter zu verbessern, können diese bevorzugt mindestens einen weiteren teilchenförmigen Feststoff, der von den vorgenannten Siliciumdioxid-Partikeln verschieden ist, enthalten. Weitere bevorzugte optionale teilchenförmige Feststoffe sind ausgewählt aus färbenden, farbgebenden, mattierenden oder glanzgebenden Pigmenten. Bevorzugte Pigmente dieser Art können anorganisch oder organisch sein. Weitere bevorzugte Pigmente weisen einen zahlenmittleren Teilchendurchmesser von 0,1 - 200 µm, bevorzugt 0,5 - 100 µm, besonders bevorzugt 1 - 50 µm und außerordentlich bevorzugt 2 - 30 µm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können. Die Farbstoffe und Farbpigmente können aus der entsprechenden Positivliste der Kosmetikverordnung bzw. der EG-Liste kosmetischer Färbemittel ausgewählt werden. Die optionalen teilchenförmigen Feststoffe können sowohl einzeln als auch im Gemisch eingesetzt werden. Die Gesamtmenge der optionalen teilchenförmigen Feststoffe beträgt bevorzugt 0,1 - 30 Gew.-%, besonders bevorzugt 0,5 - 15 Gew.-%, außerordentlich bevorzugt 1,0 - 10 Gew.-%, oder auch 2 - 4 Gew.-%, jeweils bezogen auf das Gesamtgewicht der erfindungsgemäßen Zusammensetzung.
Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, Verdickungsmittel, anfeuchtende und/oder feuchthaltende Substanzen, Fette, Öle, Wachse, Alkohole, Polyole, Polymere, Elektrolyte, organische Lösemittel oder Siliconderivate.
Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.

Besonders bevorzugte Zusammensetzungen werden ferner erhalten, wenn als Zusatz- oder Wirkstoffe Antioxidantien eingesetzt werden. Erfindungsgemäß bevorzugt enthalten die Zusammensetzungen mindestens ein Antioxidans. Als günstige, aber dennoch fakultativ zu verwendende Antioxidantien können alle für kosmetische und/oder dermatologische Anwendungen geeigneten oder gebräuchlichen Antioxidantien verwendet werden, insbesondere Tocopherol, Tocopherylacetat oder Dimethylmethoxy Chromanol, erhältlich unter "Lipochroman-6" von Lipotec.
In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 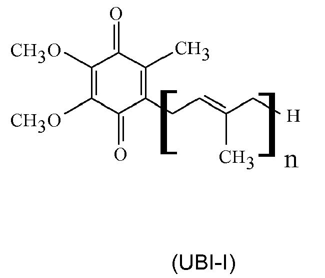 mit n = 6, 7, 8, 9 oder 10.
Besonders bevorzugt ist das Ubichinon der Formel (UBI-I) mit n = 10, auch bekannt als Coenzym Q10. Erfindungsgemäß besonders bevorzugte Zusammensetzungen sind **dadurch gekennzeichnet, dass** sie mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung, enthalten.
In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.
In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.
In einer weiteren bevorzugten Ausführungsform enthalten die erfindungsgemäßen Zusammensetzungen mindestens eine natürliche Betainverbindung. Erfindungsgemäß bevorzugte natürliche Betainverbindungen sind natürlich vorkommende Verbindungen mit der Atomgruppierung R₃N⁺-CH₂₋X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻), Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II)

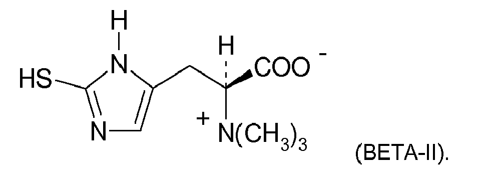

Die Herkunft der eingesetzten natürlichen Betainverbindung kann erfindungsgemäß durchaus synthetisch sein. Weiterhin können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung. Es kann auch von Vorteil sein, beliebige Enantiomerengemische, beispielsweise ein Racemat aus D-und L-Form, zu verwenden. Bevorzugte erfindungsgemäße Zusammensetzungen sind **dadurch gekennzeichnet, dass** sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.
Weitere erfindungsgemäß bevorzugte Zusammensetzungen sind **dadurch gekennzeichnet, dass** sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆-₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglycoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono-, Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.
Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.
Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion oder Hydrodispersion vor. Die Ölphase kann, insbesondere als Träger für die öllöslichen UV-Filter, mindestens ein pflanzliches oder tierisches Öl, ein Mineralöl, ein synthetisches Öl oder eine Mischung solcher Öle sein. In einer besonders bevorzugten Ausführungsform liegen die erfindungsgemäßen Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversionstemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der bevorzugten Ausführungsform als Emulsion enthalten die erfindungsgemäßen Zusammensetzungen neben dem Emulgatorgemisch d) plus e) optional mindestens eine weitere, von d) und e) verschiedene oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Polyglycerinen und Polyglycerinderivaten, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn-Salze.
Die erfindungsgemäßen Zusammensetzungen können neben dem obligatorischen Emulgatorgemisch d) plus e) mindestens einen dieser optionalen Emulgatoren bevorzugt in einer Gesamtmenge von 0,1 bis 5 Gew.-%, besonders bevorzugt 0,2 - 2 Gew.-%, bezogen auf die gesamte Zusammensetzung, enthalten.
In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher **dadurch gekennzeichnet, dass** sie in Form einer lamellare Strukturen aufweisenden Öl-in-Wasser-Emulsion vorliegen.
In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-Öl-Emulsionen formulieren, insbesondere unter Zusatz des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.
Weitere geeignete Zusatzstoffe sind Verdickungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).
Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.
Weitere bevorzugte Zusatzstoffe sind Lösungsmittel wie Ethanol, Isopropanol, Ethylenglykol, Propylenglykol, Propylenglykolmonoethylether, Glycerin und Diethylenglykol, Parfümöle, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.
In einer weiteren bevorzugten Ausführungsform liegen die erfindungsgemäßen Zusammensetzungen in einem Sprühspender oder Pumpspender verpackt vor.

Ein weiterer Gegenstand der vorliegenden Erfindung ist die Verwendung einer Mischung aus mindestens einem nicht-alkoxylierten Ölsäureester mindestens eines linearen, gesättigten C₁₀-C₂₄-Alkanols und mindestens einem nicht-alkoxylierten Sorbitan-Ölsäure-Ester zur Steigerung des Lichtschutzfaktors einer kosmetischen Zusammensetzung in Form einer öl- und wasserhaltigen Dispersion, enthaltend mindestens einen UV-Filter, ausgewählt aus öllöslichen Alkyl- und/oder Alkoxy-substituierten Dibenzoylmethanderivaten, Polysilicone-15 und wasserlöslichen Derivaten der Benzimidazolsulfonsäure sowie Mischungen dieser UV-Filter.

Ein weiterer Gegenstand der vorliegenden Erfindung ist die nicht-therapeutische, kosmetische Verwendung einer kosmetischen Zusammensetzung in Form einer öl- und wasserhaltigen Dispersion, enthaltend mindestens einen öllöslichen UV-Filter, ausgewählt aus Alkyl- und/oder Alkoxy-substituierten Dibenzoylmethanderivaten, weiterhin Polysilicone-15 und mindestens einen wasserlöslichen UV-Filter, ausgewählt aus Derivaten der Benzimidazolsulfonsäure, weiterhin mindestens einen nicht-alkoxylierten Ölsäureester mindestens eines linearen, gesättigten C₁₀-C₂₄-Alkanols und mindestens einen nicht-alkoxylierten Sorbitan-Ölsäure-Ester, wobei die Gesamtmenge der Ester d) und e) 0,4 - 15 Gew.-%, bevorzugt 1 - 10 Gew.-%, besonders bevorzugt 2 - 8 Gew.-%, außerordentlich bevorzugt 3 - 5 Gew.-%, jeweils bezogen auf das Gesamtgewicht der Zusammensetzung, beträgt, zum Schutz von Haut und Haar gegen die schädlichen Wirkungen von UV-Strahlung, insbesondere UV-A-und UV-B-Strahlung, insbesondere Sonnenstrahlung.

Bezüglich weiterer bevorzugter Ausführungsformen der erfindungsgemäßen Verwendungen gilt mutatis mutandis das zu den erfindungsgemäßen Zusammensetzungen Gesagte.

Ein weiterer Gegenstand der vorliegenden Anmeldung ist ein nicht-therapeutisches, kosmetisches Verfahren zum Schutz von Haut und Haar gegen die schädlichen Wirkungen von UV-Strahlung, insbesondere UV-A-und UV-B-Strahlung, insbesondere Sonnenstrahlung, bei dem eine kosmetische Zusammensetzung in Form einer öl- und wasserhaltigen Dispersion, enthaltend mindestens einen öllöslichen UV-Filter, ausgewählt aus Alkyl- und/oder Alkoxy-substituierten Dibenzoylmethanderivaten, weiterhin Polysilicone-15 und mindestens einen wasserlöslichen UV-Filter, ausgewählt aus Derivaten der Benzimidazolsulfonsäure, weiterhin mindestens einen nicht-alkoxylierten Ölsäureester mindestens eines linearen, gesättigten C₁₀-C₂₄-Alkanols und mindestens einen nicht-alkoxylierten Sorbitan-Ölsäure-Ester, wobei die Gesamtmenge der Ester d) und e) 0,4 - 15 Gew.-%, bevorzugt 1 - 10 Gew.-%, besonders bevorzugt 2 - 8 Gew.-%, außerordentlich bevorzugt 3 - 5 Gew.-%, jeweils bezogen auf das Gesamtgewicht der Zusammensetzung, beträgt, auf die Haut aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Die nachfolgenden Beispiele sollen den Gegenstand der vorliegenden Anmeldung verdeutlichen, ohne ihn hierauf einzuschränken. Die Zahlenwerte in den Beispielen bedeuten Gewichtsprozente, bezogen auf das Gesamtgewicht der jeweiligen Zusammensetzungen.

### Testrezepturen (ÖI-in-Wasser-Emulsionen)

| | Vergleichs-Rezeptur A | Erfindungsgemäße Rezeptur B |
|---|---|---|
| Emulsiphos 677660 | 5 | - |
| Olivem 1000 | - | 5 |
| Dibutyladipat | 5 | 5 |
| Caprylic/Capric Triglycerides | 3,5 | 3,5 |
| Safloröl | 2 | 2 |
| Behenylalkohol | 3 | 3 |
| Cetiol Sensoft | 1 | 1 |
| Vitamin E Acetat | 0,5 | 0,5 |
| Controx KS | 0,05 | 0,05 |
| Bienenwachs | 0,2 | 0,2 |
| Natriumpolyacrylat | 0,5 | 0,5 |
| Parsol 1789 | 3 | 3 |
| Parsol SLX | 3 | 3 |
| Propylparaben | 0,2 | 0,2 |
| Hexandiol-1,6 | 5 | 5 |
| Glycerin 86% | 4,5 | 4,5 |
| Sorbit 70% | 2 | 2 |
| Methylparaben | 0,2 | 0,2 |
| Tego Carbomer 140 | 0,5 | 0,5 |
| Na₃-Nitrilotriacetat - wässrig (40%) | 0,1 | 0,1 |
| Siliciumdioxid-Partikel* | 2 | - |
| 2-Amino-2-methylpropanol | 0,99 | 0,99 |
| Neo Heliopan Hydro | 3 | 3 |
| Natriumhydroxid Perlen | 0,365 | 0,288 |
| Wasser, vollentsalzt | ad 100 | ad 100 |

| | | |
|---|---|---|
| * nicht organisch oder anorganisch modifiziert, keine organische oder anorganische Beschichtung, zu mehr als 80 Gew.-% sphärische Partikel, zahlenmittlerer Partikeldurchmesser 6 - 8 µm, Ölabsorptionskapazität : 0,9 - 1,3 cm³ flüssiges Paraffin pro Gramm trockenes Siliciumdioxid (DIN 53601), erhalten durch Kieselsäure-Fällung | | |

Die beiden O/W Emulsionen A und B wurden nach Colipa Richtlinie bezüglich des in vivo LSF untersucht. Die Messungen ergaben folgende Ergebnisse für den in vivo LSF:
Rezeptur A: 15,1
Rezeptur B: 29,2

Ferner wurden die sensorischen Eigenschaften der Cremes untersucht. Hierbei konnte für die erfindungsgemäße Creme B im sensorischen Test ein verbessertes Hautgefühl im Vergleich zu Creme A festgestellt werden.

### Liste der verwendeten Rohstoffe

| Handelsname | INCI | Lieferant/Hersteller |
|---|---|---|
| Emulsiphos 677660 | Potassium Cetyl Phosphate, Hydrogenated Palm Glycerides | Symrise |
| Cosmedia SP | SODIUM POLYACRYLATE | Cognis |
| Tego Carbomer 140 | Carbomer | Evonik |
| Cetiol Sensoft | Propylheptyl Caprylate (2-Propylheptyl octanoate) | Cognis |
| Controx KS | Tocopherol, Hydrogenated Palm Glycerides Citrate | Cognis |
| Olivem 1000 | Cetearyl Olivate, Sorbitan Olivate | B & T S.R.L. (Italien) |
| Parsol 1789 | Butyl Methoxydibenzoylmethane | DSM |
| Parsol SLX | Polysilicone-15 | DSM |
| Tego Carbomer 140 | Carbomer | Goldschmidt |
| Neo Heliopan Hydro | Phenylbenzimidazol sulfonic acid | Symrise |

## Patentansprüche

1. Kosmetische Zusammensetzung in Form einer öl- und wasserhaltigen Dispersion, enthaltend
a) mindestens einen öllöslichen UV-Filter, ausgewählt aus Alkyl- und/oder Alkoxy-substituierten Dibenzoylmethanderivaten,
b) und Polysilicone-15
c) und mindestens einen wasserlöslichen UV-Filter, ausgewählt aus Derivaten der Benzimidazolsulfonsäure,
d) und mindestens einen nicht-alkoxylierten Ölsäureester mindestens eines linearen, gesättigten C₁₀-C₂₄-Alkanols,
e) und mindestens einen nicht-alkoxylierten Sorbitan-Ölsäure-Ester,
f) wobei die Gesamtmenge der Ester d) und e) 0,4 - 15 Gew.-%, bevorzugt 1 - 10 Gew.-%, besonders bevorzugt 2 - 8 Gew.-%, außerordentlich bevorzugt 3 - 5 Gew.-%, jeweils bezogen auf das Gesamtgewicht der Zusammensetzung, beträgt.

2. Kosmetische Zusammensetzung gemäß Anspruch 1, **dadurch gekennzeichnet, dass** der Ester d) ausgewählt ist aus Cetyloleat, Stearyloleat und Cetearyloleat.

3. Kosmetische Zusammensetzung gemäß Anspruch 1 oder 2, **dadurch gekennzeichnet, dass** als Ester d) ein Gemisch aus mindestens einem Ölsäureester mindestens eines linearen, gesättigten C₁₀-C₂₄-Alkanols, mindestens einem Palmitinsäureester mindestens eines linearen, gesättigten C₁₀-C₂₄-Alkanols und mindestens einem Linolsäureester mindestens eines linearen, gesättigten C₁₀-C₂₄-Alkanols enthalten ist.

4. Kosmetische Zusammensetzung gemäß Anspruch 1, 2 oder 3, **dadurch gekennzeichnet, dass** als Ester e) ein Gemisch aus mindestens einem nicht-alkoxylierten Sorbitan-Ölsäure-Ester, mindestens einem nicht-alkoxylierten Sorbitan-Palmitinsäure-Ester und mindestens einem nicht-alkoxylierten Sorbitan-Linolsäure-Ester enthalten ist.

5. Kosmetische Zusammensetzung gemäß Anspruch 3 oder 4, **dadurch gekennzeichnet, dass** die Estergemische d) und e) die Fettsäurezusammensetzung von Olivenöl aufweisen.

6. Kosmetische Zusammensetzung gemäß Anspruch 3, 4 oder 5, **dadurch gekennzeichnet, dass** der Ester d) Cetearylolivat ist und der Ester e) aus Sorbitanolivat stammt.

7. Kosmetische Zusammensetzung gemäß Anspruch 1, 2, 3, 4, 5 oder 6, **gekennzeichnet durch** ein Gewichtsverhältnis der Ester d) zu Ester e) zueinander im Bereich von 20:80 bis 80:20, bevorzugt 30:70 bis 70:30, besonders bevorzugt 40:60 bis 50:50.

8. Kosmetische Zusammensetzung gemäß Anspruch 1, 2, 3, 4, 5, 6 oder 7, **dadurch gekennzeichnet, dass** der mindestens eine UV-Filter c) ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium-, und/oder Alkanolamin-Salzen, insbesondere aus Phenylbenzimidazolsulfonsäure und dem Phenylen-1,4-bis-(2-benzimidazyl)-3,3'-5,5'-tetrasulfonsäure-bis-natriumsalz.

9. Kosmetische Zusammensetzung gemäß Anspruch 1, 2, 3, 4, 5, 6, 7 oder 8, **dadurch gekennzeichnet, dass** 4-(tert.-Butyl)-4'-methoxydibenzoylmethan, Polysilicone-15 und 2-Phenylbenzimidazol-5-sulfonsäure enthalten sind.

10. Verwendung einer Mischung aus mindestens einem nicht-alkoxylierten Ölsäureester mindestens eines linearen, gesättigten C₁₀-C₂₄-Alkanols und mindestens einem nicht-alkoxylierten Sorbitan-Ölsäure-Ester zur Steigerung des Lichtschutzfaktors einer kosmetischen Zusammensetzung in Form einer öl- und wasserhaltigen Dispersion, enthaltend mindestens einen UV-Filter, ausgewählt aus öllöslichen Alkyl- und/oder Alkoxy-substituierten Dibenzoylmethanderivaten, Polysilicone-15 und wasserlöslichen Derivaten der Benzimidazolsulfonsäure sowie Mischungen dieser UV-Filter.
